Europäisches Patentamt

European Patent Office

Office européen des brevets

(11) Veröffentlichungsnummer: **0 287 794 B1**

## EUROPÄISCHE PATENTSCHRIFT

(45) Veröffentlichungstag der Patentschrift: **15.01.92**

(51) Int. Cl.⁵: **A61M 5/315**

(21) Anmeldenummer: **88103675.0**

(22) Anmeldetag: **09.03.88**

54 **Dosierspritze.**

(30) Priorität: **25.03.87 DE 3709783**

(43) Veröffentlichungstag der Anmeldung:
**26.10.88 Patentblatt 88/43**

(45) Bekanntmachung des Hinweises auf die
Patenterteilung:
**15.01.92 Patentblatt 92/03**

(84) Benannte Vertragsstaaten:
**AT BE CH DE ES FR GB GR IT LI LU NL SE**

(56) Entgegenhaltungen:
**EP-A- 0 106 578**
**DE-U- 8 704 415**
**GB-A- 1 007 328**
**GB-A- 2 109 690**
**US-A- 3 327 904**

(73) Patentinhaber: **Blendax GmbH**
**Rheinallee 88**
**W-6500 Mainz 1(DE)**

(72) Erfinder: **Bartsch, Klaus**
**Friedrich-Ebert-Strasse 13**
**W-6501 Budenheim(DE)**

(74) Vertreter: **TER MEER - MÜLLER - STEINMEI-**
**STER & PARTNER**
**Artur-Ladebeck-Strasse 51**
**W-4800 Bielefeld 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Dosierspritze mit einem zylindrischen Spritzenkörper, der an einem sich verjüngenden Ende eine Ausgabeöffnung und am anderen Ende eine stufenförmige zylindrische Erweiterung und zwei sich gegenüberliegende Griffflügel aufweist, unter denen sich jeweils eine Aussparung befindet, wobei vom erweiterten Ende in den zylindrischen Spritzenkörper ein Kolben eingeschoben ist, an den sich zwei voneinander getrennte, jeweils ein Innengewinde aufweisende Kolbenbacken anschließen, mit einem ein Außengewinde und einen Längsnocken aufweisenden Dosierstempel des in den zweiteiligen, in der Erweiterung mit einem Spalt zueinander angeordneten Kolbenbacken drehbar angeordnet ist, wobei jede Kolbenbacke einen in die Aussparung der Erweiterung einführbaren Fixiervorsprung aufweist.

Bei einer solchen bekannten, vorbenutzten Dosierspritze wird nach dem Befüllen des Spritzenkörpers der Kolben eingeschoben, wonach die zweigeteilten Kolbenbacken, die abgefederte Fixiervorsprünge aufweisen, in die zylindrische Erweiterung eingelegt und durch Eindrehen des Dosierstempels in das Gewinde der Gewindebacken dadurch fixiert werden, daß dabei die Fixiervorsprünge in die Aussparungen der zylindrischen Erweiterung eingeführt werden.

Der geschilderte Zusammenbau der Dosierspritze, der zudem bei gefülltem Spritzenkörper durchgeführt werden muß, ist umständlich und zeitaufwendig und birgt die Gefahr der Verunreinigung oder Verschüttung des Füllguts der Spritze in sich.

Aufgabe der Erfindung ist es, eine Dosierspritze der eingangs genannten Art dahingehend verbessern, daß der Zusammenbau der Dosierspritze vereinfacht und so die Gefahr eines Verschüttens oder Verunreinigens des Füllguts vermieden wird.

Diese Aufgabe wird durch eine Dosierspritze mit den Merkmalen des Anspruchs 1 gelöst.

Nachstehend wird die Erfindung anhand eines Ausführungsbeispiels unter Angabe von vorteilhaften Variationen unter Bezug auf Zeichnungen näher erläutert. Es zeigt:

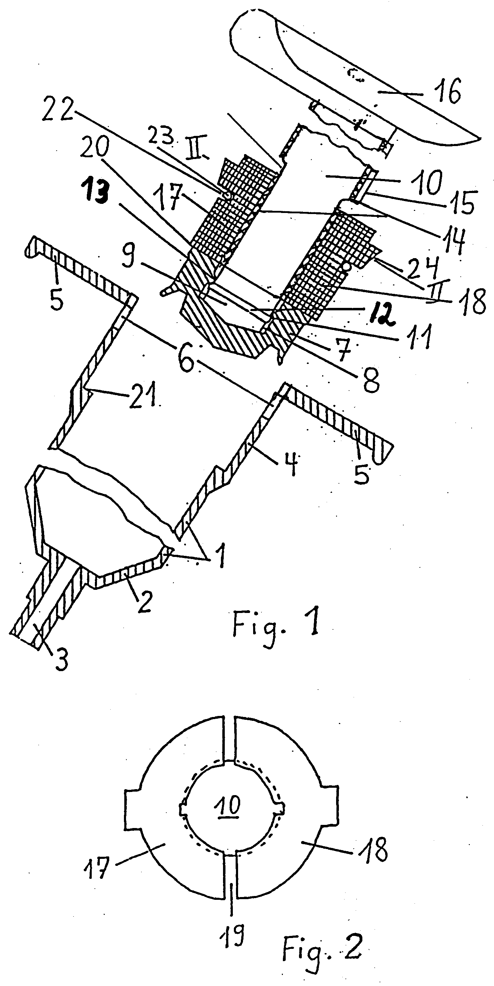

Fig. 1 eine Dosierspritze mit vorkomplettierten Teilen vor dem Zusammenbau in vergrößertem Maßstab,

Fig. 2 einen Schnitt gemäß Linie II-II in Fig. 1

Eine Dosierspritze weist einen zylindrischen Spritzenkörper 1 auf. Der Spritzenkörper 1 weist ein sich verjüngendes Ende 2 mit einer Ausgabeöffnung 3 auf. Das verjüngte Ende 2 kann für das Aufsetzen eines Verschlusses und einer Spritznadel (nicht gezeigt) oder einer anderen Ausgabedüse, die dem jeweiligen Verwendungszweck der Dosierspritze angepaßt ist, gestaltet sein. Am anderen Ende weist der Spritzenkörper 1 eine stufenartige zylindrische Erweiterung 4 und an deren freiem Ende zwei sich gegenüberliegende Griffflügel 5 auf. Der Spritzenkörper 1 mit der Ausgabeöffnung 3 der stufenartigen zylindrischen Erweiterung 4 und den Griffflügeln 5 ist einstückig hergestellt. Unter jedem Griffflügel 5 ist eine Aussparung 6 in der Wandung der stufenartigen zylindrischen Erweiterung 4 ausgeführt.

Zur Anordnung im zylindrischen Spritzenkörper 1 ist ein Kolben 7 vorgesehen. Der Kolben 7 weist ein zentrales Blindloch 8 auf, in das ein entsprechend gestalteter Vorsprung 9 eines Dosierstempels 10 mittels einer Schnappverbindung 11 einrastbar einführbar ist. Durch die Schnappverbindung 11 wird eine feste Verbindung zwischen Kolben 7 und Dosierstempel 10 erreicht. Die Schnappverbindung 11 kann auf eine beliebige bekannte Art, z.B. durch das Zusammenwirken von einer oder gegebenenfalls mehreren Nuten mit einem oder gegebenenfalls mehreren Vorsprüngen gestaltet sein. Im Ausführungsbeispiel weisen das Blindloch 8 und der Vorsprung 9 jeweils einen ringförmigen gerundeten Vorsprung 12 bzw. 13 auf, die so gegeneinander versetzt angeordnet sind, daß, wenn das freie Ende des Vorsprungs 9 am Boden des Blindlochs 8 anliegt, der ringförmige gerundete Vorsprung 12 des Blindlochs 8 in Einschubrichtung vor dem ringförmig gerundeten Vorsprung 13 des Vorsprungs 9 liegt und diesen an einem Herausrutschen aus dem Blindloch 8 hindert.

Im Anschluß an den Vorsprung 9 weist der Dosierstempel 10 ein Schraubgewinde 14, einen vorspringenden Längsnocken 15 sowie einen Griff 16 am Ende auf. In der stufenartigen zylindrischen Erweiterung 4 sind zwei etwa halbkreisförmig gestaltete Kolbenbacken 17,18 mit einem geringen Spalt 19 zueinander angeordnet. Die Kolbenbacken 17,18 weisen Innengewinde auf, die dem äußeren Schraubgewinde 14 des Dosierstempels 10 angepaßt sind. Die Kolbenbacken 17,18 stützen sich mit ihrer unteren Kante auf der Stufe 21 der stufenartigen Erweiterung 4 ab und bilden mit ihren oberen Flächen eine Ebene mit der oberen Fläche der Griffflügel 5. In ihrer im Querschnitt halbkreisförmigen Außenfläche weisen die Kolbenbacken 17,18 eine Nut 22 auf, in die ein die Kolbenbacken 17,18 zusammenhaltender elastischer O-Ring 23 versenkt eingelegt ist. Die Kolben backen 17,18 weisen jeweils einen keilförmigen Vorsprung 24 an ihrer Außenfläche auf, die zum Eingriff in die unterhalb der Griffflügel 5 ausgeführten Aussparungen 6 bestimmt und dementsprechend ausgestaltet und angeordnet sind. Der Abstand der oberen den Griffflügeln 5 benachbarten Kanten der keilförmigen Vorsprünge 24 ist im am Gewinde des Dosierstempels 10 anliegenden Zustand etwas größer als der lichte Durchmesser der zylindrischen Erweiterung 4 gewählt, so daß die keilförmigen Vorsprünge unter Ausnützung ihrer elastischen Deformation durch den zylindrischen geschlossenen Teil der zylindrischen Erweiterung 4 durchgeschoben werden und sich erst in den Aussparungen 6 wieder entspannen können und dadurch eine feste Verriegelung des Dosierstempels mit dem darauf aufgesetzten Kolben im Spritzenkörper garantieren.

Es besteht auch die Möglichkeit, die keilförmigen Vorsprünge 24 nicht wie beschrieben starr mit den Kolbenbacken 17,18 verbunden auszuführen, sondern federnd an den Gewindebacken 17,18 anzuordnen, z.B. durch Ausführung eines Spaltes (nicht dargestellt), der bewirkt, daß die Vorsprünge 24 mittels eines durch Materialverdünnung erzeugten Scharniers an den Gewindebacken 17,18 befestigt sind. In diesem Fall brauchen die Abmessungen der Vorsprünge 24 nicht so genau auf den lichten Durchmesser der zylindrischen Erweiterung 4 abgestimmt sein, jedoch erhöhen sich die Kosten für die Spritzformen zur Herstellung der Kolbenbacken 17,18.

Durch die Erfindung ist es nun möglich, nach oder vor dem Aufschieben des Kolbens 7 auf den Dosierstempel 10 und deren gegenseitiger Befestigung mittels der Schnappverbindung 11 die Kolbenbacken 17 und 18 einander gegenüberliegend mit ihren Innengewinden in das Außengewinde 14 des Dosierstempels 10 eingreifend auf dem Dosierstempel 10 anzuordnen und mittels des in ihre Nuten 22, die zusammen einen geschlossenen Kreis bilden, eingelegten O-Rings 23 in ihrer gegenseitigen Lage zusammenzuhalten. Hierdurch ist es möglich, einen vorkomplettierten Kolbenstempel zu erhalten, der nach Befüllen des Spritzenkörpers 1 lediglich in das obere Ende des Spritzenkörpers 1 derart eingeführt werden muß, daß die keilförmigen Vorsprünge 24 der Kolbenbacken 17,18 in die Aussparungen 6 einrasten und so den Kolbenstempel im Spritzenkörper 1 fixieren. Durch Drehen des Dosierstempels 10 im Gewinde der Kolbenbacken 17,18 braucht dann nur noch de Nullstellung eingestellt werden. Durch die Erfindung wird erreicht, daß der Zusammenbau der Einzelteile des Kolbenstempels unter erleichterten Bedingungen erfolgen kann und daß die Arbeit mit dem gefüllten Spritzenkörper auf ein Minimum begrenzt werden kann, so daß die Gefahr des Verschüttens und des Eindringens von Verunreinigungen minimiert wird.

## Patentansprüche

1. Dosierspritze mit einem zylindrischen Spritzenkörper (1), der an einem sich verjüngenden Ende (2) eine Ausgabeöffnung (3) und am anderen Ende eine stufenförmige zylindrische Erweiterung (4) und zwei sich gegenüberliegende Grifflügel (5) aufweist, unter denen sich jeweils eine Aussparung (6) befindet, mit einem vom erweiterten Ende in den zylindrischen Spritzenkörper (1) eingeschobenen Kolben (7), an den sich zwei voneinander getrennte, jeweils ein Innengewinde aufweisende Kolbenbacken anschließen, mit einem ein Außengewinde (14) und einen Längsnocken (15) aufweisenden Dosierstempel (10), der in den zweiteiligen, in der Erweiterung (4) mit einem Spalt (19) zueinander angeordneten Kolbenbacken (17, 18) drehbar angeordnet ist, wobei jede Kolbenbacke (17 bzw. 18) einen in die Aussparung (6) der Erweiterung (4) einführbaren Fixiervorsprung (24) aufweist, **dadurch gekennzeichnet,** daß der Kolben (7) mit dem Dosierstempel (19) mittels einer Schnappverbindung (11) verbunden ist und daß die Kolbenbacken (17, 18) eine außen liegende Nut (22) aufweisen, in der ein beide Gewindebacken (17, 18) zusammenhaltender O-Ring (23) versenkt eingelegt ist.

2. Dosierspritze nach Anspruch 1, dadurch gekennzeichnet, daß die Fixiervorsprünge (24) starr mit den Kolbenbacken (17, 18) verbunden und derart dimensioniert und/oder gestaltet sind, daß sie bei Anordnung der Kolbenbacken auf dem Dosierstempel (19) in die zylindrische Erweiterung einschieb- und in die Aussparungen (6) einrastbar sind.

## Claims

1. Dosing syringe with a cylindrical syringe body (1) which has an outlet opening (3) at one tapering end (2) and a step-shaped cylindrical enlargement (4) at the other end and two mutually opposed wings (5) beneath which there is a respective recess (6) with a piston (7) which is inserted from the enlarged end into the cylindrical syringe body (l) and to which there are attached two piston jaws which are separated from one another and each have an internal thread, with a dosing plunger (10) which has an external thread (14) and a longitudinal cam (15) and is rotatably arranged in the two-part piston jaws (17, 18) arranged in the enlargement (4) with a gap (19) from one another, wherein each piston jaw (17 and 18) has a fixing projection (24) which can be introduced into the recess (6) in the enlargement (4), characterised in that the piston (7) is connected to the dosing plunger (19) by means of a snap connection (11) and in that the piston jaws (17, 18) have an externally located groove (22) in which an O-ring (23) holding the two threaded jaws (17, 18) together is embedded.

**2.** Dosing syringe according to claim 1, characterised in that the fixing projections (24) are rigidly connected to the piston jaws (17, 18) and are dimensioned and/or designed such that they can be inserted in the cylindrical enlargement and are capable of catching in the recesses (6) if the piston jaws are arranged on the dosing plunger (19).

**Revendications**

**1.** Seringue de dosage comportant un corps cylindrique (1), qui présente à une extrémité rétrécie (2), une ouverture de sortie (3) et, à l'autre extrémité, une partie élargie cylindrique étagée (4) et deux ailettes opposées de préhension (5), entre lesquelles est situé respectivement un évidement (6), comportant un piston (7) inséré à partir de l'extrémité élargie dans le corps cylindrique (1) de la seringue et auquel se raccordent deux mâchoires séparées l'une de l'autre, présentant chacune un taraudage, et un piston de dosage (10), présentant un filetage extérieur (14) et une came longitudinale (15) et est monté rotatif dans les mâchoires de piston (17,18) réalisées en deux éléments et disposées dans la partie élargie (4) en étant séparées par une fente (19), chaque mâchoire (17 ou 18) présentant une partie saillante de fixation (24) pouvant être insérée dans l'évidement (6) de la partie élargie (4), caractérisée par le fait que le piston (7) est raccordé au piston de dosage (19) au moyen d'une liaison à encliquetage brusque (11), et que les mâchoires de piston (17,18) présentant une gorge extérieure (22), dans laquelle est inséré en renfoncement un joint torique (23) maintenant assemblées les deux mâchoires filetées (17,18).

**2.** Seringue de dosage selon la revendication 1, caractérisée en ce que les parties saillantes de fixation (24) sont raccordées rigidement aux mâchoires de piston (17,18) et sont dimensionnées et/ou conformées de telle sorte que, lors du montage des mâchoires de piston sur le piston de dosage (10), ces parties saillantes peuvent être insérées dans la partie élargie cylindrique et être encliquetées dans les évidements (6).

Fig. 1

Fig. 2